(19) Europäisches Patentamt

European Patent Office

Office européen des brevets

(11) Publication number: **0 358 399 B1**

## (12) EUROPEAN PATENT SPECIFICATION

(45) Date of publication of patent specification: 04.08.93    (51) Int. Cl.5: **A61F 2/36**

(21) Application number: **89308760.1**

(22) Date of filing: **30.08.89**

(54) **Load sharing femoral hip implant.**

(30) Priority: **31.08.88 US 238913**

(43) Date of publication of application:
**14.03.90 Bulletin 90/11**

(45) Publication of the grant of the patent:
**04.08.93 Bulletin 93/31**

(84) Designated Contracting States:
**AT BE CH DE ES FR GB GR IT LI LU NL SE**

(56) References cited:
**EP-A- 0 216 489**
**DE-A- 2 247 721**
**FR-A- 2 438 469**

**MEDICAL & BIOLOGICAL ENGINEERIN0 &
COMPUTING, vol. 26, no. 1, January 1988,
pages 38-45, IFMBE, Stevenage, Herts, GB;
J.A. ENGELHARDT et al. : "Effect of femoral
component section modulus on the stress
distribution in the proximal human femur"**

(73) Proprietor: **BOEHRINGER MANNHEIM CORPO-
RATION**
**P.O. Box 5 05 28**
**Indianapolis Indiana 46250(US)**

(72) Inventor: **Kelman, David C.**
**107 Southfield Road**
**Winona Lake, IN 46590(US)**
Inventor: **Smith, Todd**
**2006 N. Robb Road**
**Warsaw, IN 46580(US)**

(74) Representative: **Cook, Anthony John et al**
**D. YOUNG & CO. 10, Staple Inn**
**London, WC1V 7RD (GB)**

# Description

The present invention relates generally to a femoral hip prosthesis and, more particularly, to a femoral component which can be stress tailored to optimally share the load with the femur in which it is implanted.

Based on the precepts of Wolff's Law which states that bone tissue will remodel in direct relation to the stress applied to it, it is desirable to stress bone at an optimal level to minimize and control remodeling after THR (total hip replacement) arthroplasty. Usually some degree of proximal femur bone remodeling accompanies total hip replacement. Due to mechanical stiffness, metallic implants typically stress protect the proximal bone to some extent. In patients with relatively large intramedullary canals which require a large diameter implant for optimal fit, stress protection may be particularly troublesome. In the most extreme case, the proximal femoral bone may resorb to a small fraction of its original mass, possibly causing a loss of support of the implant or implant breakage. It is unfortunate that implant flexural stiffness increases at an exponential rate, typically at powers between two and four, depending upon implant geometry, relative to linear increases in implant dimension. Further aggravating the situation is the fact that there is little correlation between the size of the patient and the diameter of the intramedullary canal. That is, a small, relatively light person may have a femur with a large diameter canal and a much larger person may have a femur with a smaller diameter canal. Therefore, it is desirable to produce an implant, especially a larger diameter implant, with greatly reduced stiffness in relation to its mass.

This can be accomplished in several ways. For example the use of materials which are inherently less stiff, that is, possess a lower flexural modulus might be considered. Thus, the use of titanium alloy or a carbon fiber reinforced polymer composite in lieu of the stiffer cobalt-chrome alloy might be considered. An implant can also be hollowed out. This method is marginally effective, however, due to the fact that the centrally located material contributes little to the stiffness of the implant. For example, if an implant with a round stem of 16mm diameter is hollowed to a wall thickness of only 2mm, the resulting decrease in flexural stiffness is only 32% while the decrease in mass is 56%. Interestingly, a 16mm diameter stem is 6.5 times stiffer than the 10mm diameter stem. Morscher and Dick reported on nine years of clinical experience with a so-called "isoelastic" shaft prosthesis manufactured using polyacetal resin to transmit forces from the pelvis through the femoral head and neck into the femur in their paper: "Cementless Fixation of 'Isoelastic' Hip Endoprostheses Manufactured from Plastic Materials", Clinical Orthopaedics, June, 1983, Volume 176, pages 77-87. They stated: "The optimal fixation of an implant depends mainly on its design and material. The insertion of an artificial joint induces remodeling of the bony structures. If stability is not achieved, the implant sooner or later will loosen. The elasticity, and consequently the deformation, of an implant depend on the elastic modulus of the material and on the prosthetic design. By adjusting the physical characteristics of the foreign material to that of the bone tissue, as well as the design of the prosthesis to the femoral shaft, the entire system would have the same elasticity as a normal femur. A more elastic hip endoprosthesis also may act as a shock absorber during walking, particularly in the heel/strike and toe/off phases."

They proceeded to explain that this was the concept of the "isoelastic" hip endoprosthesis manufactured by Robert Mathys and implanted in 1973. In this instance, the prosthesis was composed of polyacetal resin which has an elasticity modulus approaching that of bone tissue, good durability, and tenacity for highly stressed components in combination with good tissue tolerance. To achieve the acquired structural strength in the neck portion, the component was reinforced by a metallic core that was tapered toward the tip to increase the elasticity of the stem, thereby allowing the stem of the prosthesis to follow the deformation of the bone. In commenting on the design, the authors further stated: "Isoelasticity implies the optimum approximation of the physical characteristics of an implant to those of the bone. An ideal isoelasticity, however, can never be achieved, since bone is anisotropic and the alloplastic materials used for joint arthroplasty show isotropic properties. In addition, there is no adaptation of the structures to the forces acting on the hip, as in the case in viable bone. Moreover, the variety of individual forms and strengths of human bone can never be imitated by an artificial joint. Use of more elastic materials, however, should avoid the disadvantages of the rigid materials used to date."

U.S. Patent No. 4,287,617 to Tornier discloses a hip prosthesis with a femoral stem which provides a measure of the elasticity spoken of by Morscher and Dick. A transverse section of the Tornier stem is in the form of a substantially rectangular tube of which one of the small sides is virtually cut away so as to leave a very large slot. The C-shaped section thus obtained is said to exhibit excellent transverse elasticity which facilitates the positioning of the pin in the medullary cavity by insertion. Other stated advantages are that the pin is not as heavy as solid designs, and that the cavity encourages bone growth.

An alternative approach to the foregoing is the subject of commonly assigned U.S. Patent Number 4 808 186 to co-inventor Todd S. Smith entitled "Controlled Stiffness Femoral Hip Implant". In that construction, the medial side of the length of the implant is milled out to form a channel shaped stem cross section. The amount of material removed determines the resulting decrease in stiffness of the implant while the outside geometry remains substantially unchanged with the exception of the open channel on the medial side of the implant. The resulting longitudinal channel lies generally in the coronal plane when the stem is in the implanted condition. The depth of the channel is variable between the proximal and distal ends of the femoral implant so as to affect the mass moment of inertia at any given location along a length of the stem to thereby achieve an optimal stem flexibility. That is, the stem is so formed that at specified locations along its length, it substantially correlates to the flexibility of the femur itself.

In DE-A-2247721, there is disclosed a hip joint prosthesis having a first co-operating member securable to a first bone having an intramedullary canal and a second co-operating member securable to a second bone, the members being interengageable and relatively moveable, the first member having an elongate stem having a longitudinal axis lying generally in a coronal plane and being receivable in the intramedullary canal of the first bone, the stem extending between proximal and distal ends and having at least one reduced mid-stem section so as to affect the mass movement of inertia at any given location along the length of the stem thereby to achieve an optimal stem flexibility. However, the stem of DE-A-2247721 is disadvantageous in that the cross section thereof varies along the mid-stem portion. Accordingly, the mid-stem portion is prone to damage on insertion of the first member into the intramedullary canal.

According to a first aspect of the invention, there is provided a component of an artificial joint for replacing a damaged natural joint in a skeletal structure of a body which includes a prosthesis having a first cooperating member securable to a first, long, bone having an intramedullary canal and a second cooperating member securable to a second bone, the first and second cooperating members being interengaging and relatively movable to permit relative movement between the first and second bones, the component comprising:

an elongated stem having a longitudinal axis lying generally in a coronal plane and being integral with the first cooperating member and receivable in the intramedullary canal of the first bone, said stem extending between proximal and distal ends and having a reduced mid-stem section in a region spaced from both said proximal and said distal ends, so as to affect the mass moment of inertia at any given location along the length of said stem to thereby achieve an optimal stem flexibility, characterised in that the mid-stem section is substantially uniform in cross section along the length thereof.

According to a second aspect of the invention, there is provided an artificial joint for replacing a damaged natural joint in a skeletal structure of a body comprising:

a cup shaped socket member fixable to a first bone of the joint;

a ball member rotatably engageable with said socket member;

a mounting member including an elongated stem for securing said ball member to a second bone separate from the first bone, said second bone being a long bone with an intramedullary canal, said stem having a longitudinal axis lying generally in a coronal plane and being receivable in the intramedullary canal of the second bone, said stem extending from a proximal end adjacent said ball member to a distal end distant from said ball member, and having a reduced mid-stem section in a region spaced from both said proximal and said distal ends, said stem defining proximal, intermediate and distal segments, each segment having a predetermined length terminating at respective proximal and distal ends, said proximal end of the proximal segment being integral with said ball member and said distal end of the distal segment defining a terminal end of the stem; and

said distal end of the proximal segment and the proximal end of the distal segment being directly adjacent to and forming a tapered junction with said proximal and distal ends of the intermediate segment, respectively;

characterised in that the cross sectional area of the intermediate segment is substantially uniform in cross section over the length thereof thereby to affect the mass moment of inertia at any given location along the length of said stem and thereby achieve an optimal stem flexibility.

Briefly stated, the central portion of the length of the implant is machined to reduce its outside dimension such that it has a smaller cross section. The amount of material removed determines the resulting decrease in stiffness of the implant.

Because of the reduction of the moment of inertia of the implant stem, it is more flexible. It also experiences higher stem stresses upon loading of the implant due to its reduced section size. Therefore, a careful balance must be achieved between the amount of material removed from the stem and the expected stress levels expected by the particular size implant.

The material removed from the periphery of the mid-stem region of the implant may be in a uniform fashion or preferentially. The amount of material comprising the reduced section of the implant may be variable between the proximal and distal ends of the femoral implant so as to affect the mass moment of inertia at any given location along a length of the stem to thereby achieve an optimal stem flexibility. That is, the stem is so formed that at specified locations along its length, it may substantially correlate to the flexibility of the femur itself.

However, it is desirable that when the mid-stem section is formed, the resultant dimension of the mid-stem be no smaller than 25% of the original cross sectional dimension assuming the cross sectional shape is approximately round. Reasons supporting this desirable relationship include the fact that the stem may otherwise deform due to impact forces needed to insert the implant into the femur. Indeed, to preclude this potential difficulty, it is preferable that the final cross sectional area will be no less than 25% of the dimension of a comparable full sized non-contoured implant stem.

The femoral stem exhibiting the qualities of the invention may be composed of any of the common materials generally employed for implants including titanium, titanium alloy, cobalt-chromium alloy, and composite materials. However, the use of ceramics and sintered powdered metal constructions may also be considered.

The reduced mid-stem section itself may be formed during a molding process or by mechanical or chemical milling procedures, or in any other suitable fashion.

Also, according to the invention, it is considered that there would be a standard size range of stems, perhaps seven to ten different sizes varying in outer diameter, length, dimension of the reduced mid-stem section, the amount of the taper from the proximal to the distal ends of the stem. The closest sizes would be determined radiographically prior to surgery, although the final size decided upon for implanting could be finally chosen during surgery.

Other and further features, advantages, and benefits of the invention will become apparent from the following description taken in conjunction with the following drawings. It is to be understood that the foregoing general description and the following detailed description are exemplary and explanatory but are not to be restrictive of the invention. The accompanying drawings which are incorporated in and constitute a part of this invention, illustrate one of the embodiments of the invention and, together with the description, serve to explain the principles of the invention in general terms. Like numerals refer to like parts throughout the disclosure.

Fig. 1 is a side elevation view, certain parts being cut away and shown in section, of a hip prosthesis, including a femoral component embodying the invention;

Fig. 2 is a cross section view taken generally along line 2--2 in Fig. 1;

Fig. 3 is a bar graph indicating relative stiffness of a series of stems of varying diameters for femoral components which are currently available commercially;

Fig. 4 is a cross section view of the stem of a femoral component having a circular cross section and awaiting modification according to the invention;

Fig. 5 is, in part, a schematic side elevation view, partly in section, of a femoral component embodying the invention and, in part, a graph presenting the section properties at various locations on the implant; and

Fig. 6 is a graph illustrating the relative flexibility of the series of femoral components which were presented in Fig. 5.

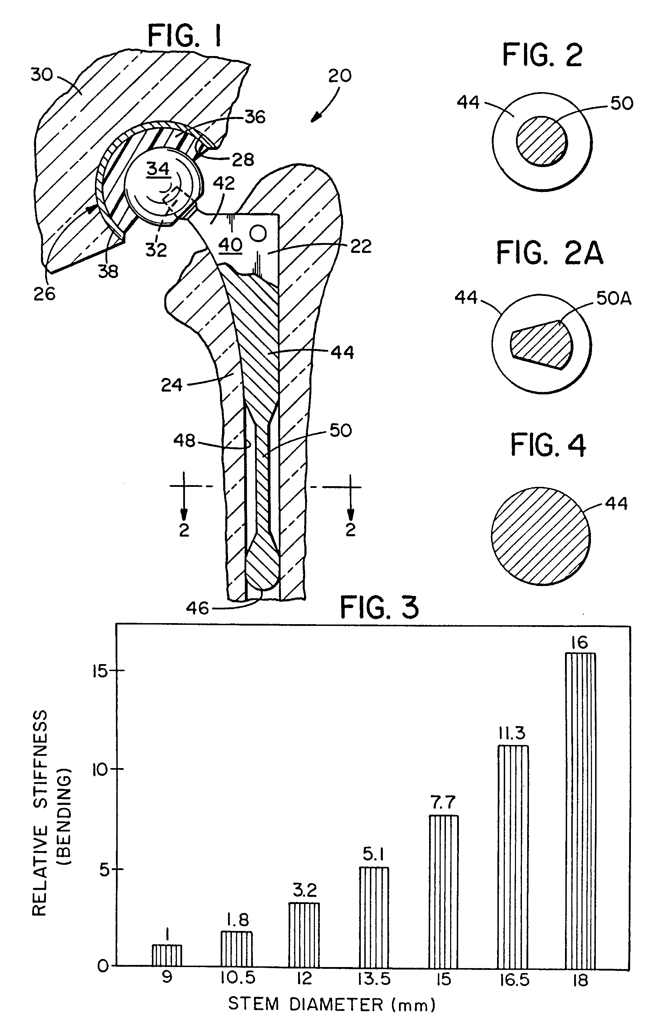

Turn now to the drawings, and initially to Fig. 1, which illustrates a hip prosthesis 20 which embodies the invention. As illustrated, a femoral component 22 is suitably implanted in the femur 24 and is cooperatively engaged with an acetabular component 26. The latter component is suitably implanted in the acetabulum 28 of the pelvis 30. In customary fashion, the femoral component 22 has a taper 32 at its extreme proximal end adapted to fittingly receive thereon a ball 34. In turn, the ball is rotatably engaged with a bearing 36 of the acetabular component 26 which may be supported in a metal cup 38 which is generally fixed to the pelvis 30. The femoral component 22 further includes a shoulder 40, with the taper 32 being joined to the shoulder via a neck 42. A stem 44 extends away from the shoulder 40 to a distal or tip end 46.

In a customary manner, the stem 44 is received in the intramedullary canal 48 of the femur 24. Stem 44 is formed with a reduced mid-section 50 which lies generally in the coronal plane of the body of the person in whom the prosthesis is implanted. The purpose of the reduced mid-section 50 (see especially Fig. 2) between the proximal and distal ends of the femoral component 22 is to affect the mass moment of inertia of the femoral component along the length of the stem 44 to thereby achieve an optimal stem flexibility. The shape of the cross section 50 may vary from stem to stem, including shapes asymmetrical in longitudinal section as desired, in order to achieve optimal stem flexibility. A stem cross section as indicated at 50A in Fig. 2A is generally representative of such a shape.

It was previously mentioned as being unfortunate that implant flexural stiffness increases at an exponential rate, typically at powers between two and four, depending upon implant geometry, relative to linear increases in implant dimension.

Graphic proof of this statement is presented in Fig. 3 which is a bar graph indicating relative stiffness of a series of stems of varying diameters which are currently available for implanting. It is noteworthy that the 18 millimeter diameter stem exhibits 16 times the stiffness of the 9 millimeter diameter stem. The invention serves to avoid this exponential increase and limits the increase in stiffness to an approximately linear relationship with increasing stem diameter.

It will be appreciated that femoral hip implants are subjected predominately to a bending mode of loading based on biomechanical analyses. This loading give rise to the highest stem stresses according to the formula:

$$S_{max} = Mc/I$$

where $S_{max}$ is the maximum stress at any location of interest along the stem; M is the bending moment imparted to the structure at the particular location of interest; c is the distance form the neutral axis to the location of interest; and I is the mass moment of inertia, a geometrical consideration.

If the maximum allowable stress based on material limitations is known and if the loading condition of the implant based on biomechanical analyses is known, one can then solve for the necessary moment of inertia via the rearrangement of the above equation, as follows:

$$I = Mc/S_{max}$$

The cross-sectional area of the reduced mid-stem section shall not be less than 25% of the unmodified stem cross-sectional area, which is to say that the original cross-sectional area shall not be reduced by more than 75%, so that the forces required to introduce the implant into the femur do not deform the implant.

Furthermore, stiffness of the desired implant at any given location along its length is a known quantity. This is determined from clinical experience. Stiffness is proportional to the moment of inertia, I, and therefore increases in proportion to the fourth power of the diameter of the stem. However, according to the invention, this increase would be limited to a fraction of what it would be for a solid implant and this fractional increase is achieved by means of the reduced mid-stem section.

With the aid of Fig. 4, it should be clear that

$$I_{implant\ w/reduced\ section} = I_{circle} - I_{reduced\ section}$$

Thus, $I_{implant\ w/reduced\ section}$ is determined for various reduced section dimensions used to satisfy the aforementioned equation, namely:

$$S_{max} = Mc/I$$

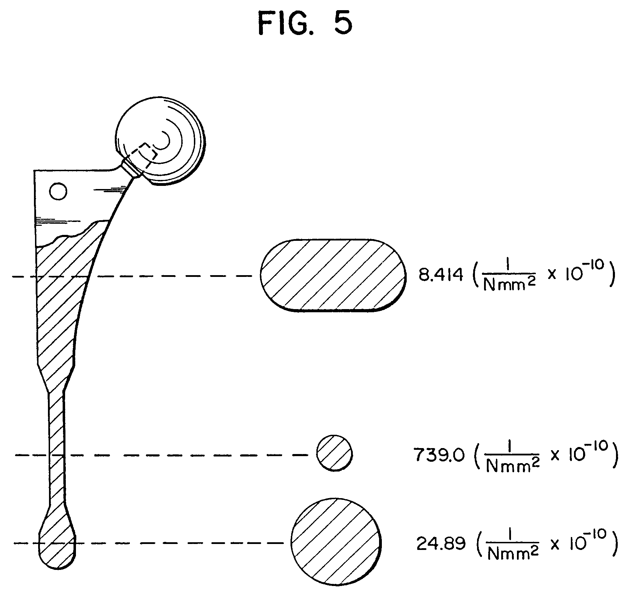

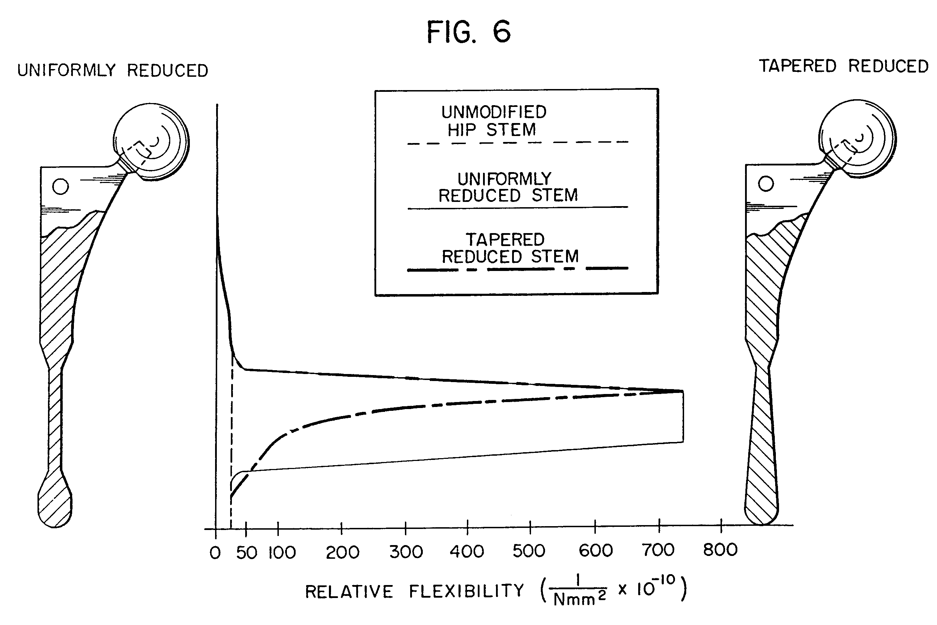

As was previously explained, the primary thrust of the invention is to prevent stress shielding at the proximal end of the femur 24 and, toward this end, to impart more stress and more strain into the femur. This desired result has been achieved as is seen in Fig. 5 which is illustrative of the relative flexibility of the test implant 58 at three of the cross sectional locations presented in the Fig. 6 graph.

While various embodiments of the invention have been disclosed in detail, it should be understood by those skilled in the art that various other modifications may be made to the illustrated embodiments without departing from the invention.

## Claims

1. A component of an artificial joint (20) for replacing a damaged natural joint in a skeletal structure of a body which includes a prosthesis having a first cooperating member (22) securable to a first, long, bone (24) having an intramedullary canal (48) and a second cooperating member (26) securable to a second bone (30), the first (22) and second (26) cooperating members being interengaging and relatively movable to permit relative movement between the first (24) and second (30) bones, the component comprising:

   an elongated stem (44) having a longitudinal axis lying generally in a coronal plane and being integral with the first cooperating member (22) and receivable in the intramedullary canal (48) of the first bone (24), said stem (44) extending between proximal (40) and distal (46) ends and having a reduced mid-stem section (50) in a region spaced from both said proximal and said distal ends, characterised in that the mid-stem section (50) is substantially uniform in cross section along the length thereof, so as to affect the mass moment of inertia at any given location along the length of said stem (50) to thereby achieve an optimal stem flexibility.

2. An artificial joint for replacing a damaged natural joint in a skeletal structure of a body comprising:

   a cup shaped socket member (36) fixable to a first bone (30) of the joint;

   a ball member (34) rotatably engageable with said socket member (36);

a mounting member (22) including an elongated stem (44) for securing said ball member (34) to a second bone (24) separate from the first bone, said second bone being a long bone with an intramedullary canal (48), said stem having a longitudinal axis lying generally in a coronal plane and being receivable in the intramedullary canal (48) of the second bone (24), said stem extending from a proximal end adjacent said ball member to a distal end distant from said ball member, and having a reduced mid-stem section in a region spaced from both said proximal and said distal ends, said stem (44) defining proximal (40), intermediate (50) and distal (46) segments, each segment terminating at respective proximal and distal ends, said proximal end of the proximal segment (40) being secured to said ball member (34) and said distal end of the distal segment (46) defining a terminal end of the stem; and

said distal end of the proximal segment (40) and the proximal end of the distal segment (46) being directly adjacent to and forming a tapered junction with said proximal and distal ends of the intermediate segment (50), respectively;

characterised in that the cross sectional area of the intermediate segment (50) is substantially uniform in cross section over the length thereof thereby to affect the mass moment of inertia at any given location along the length of said stem (44) and thereby achieve an optimal stem flexibility.

3. A component according to claim 1 or a joint according to claim 2 wherein said mid-stem section (50) is symmetrical in cross section.

4. A component according to claim 1 or a joint according to claim 2 wherein said mid-stem section (50) is asymmetrical in cross section.

5. A component according to claim 1 or a joint according to claim 2 wherein the cross sectional area of said mid-stem section (50) may be reduced by up to 75% of the area of a comparable full-sized non-contoured implant stem.

6. A component according to claim 1 or a joint according to claim 2 which is composed of any one of titanium, titanium alloy, cobalt-chromium alloy, and composite materials.

7. An artificial joint according to claim 2 wherein the first bone is the pelvis; and wherein the second bone is the femur; and wherein said ball member and said stem are parts of a femoral component of a hip prosthesis.

## Patentansprüche

1. Bestandteil eines künstlichen Gelenkes für den Ersatz eines beschädigten natürlichen Gelenkes in einer Skelettstruktur eines Körpers mit einer Prothese mit einem ersten zusammenwirkenden Teil (22), das an einem ersten langen Knochen (24) mit einem Intramedullarkanal (48) befestigbar ist, und einem zweiten zusammenwirkenden Teil (26), das an einem zweiten Knochen (30) befestigbar ist, wobei das erste (22) und das zweite (26) zusammenwirkende Teil ineinandergreifen und in Bezug aufeinander bewegbar sind, um eine Relativbewegung zwischen dem ersten (24) und dem zweiten (30) Knochen zu erlauben, wobei der Bestandteil einen länglichen Stiel (44) mit einer Längsachse, die allgemein in einer Koronarebene liegt und aus einem Stück mit dem ersten zusammenwirkenden Teil (22) besteht und von dem Intramedullarkanal (48) des ersten Knochens (24) aufnehmbar ist, umfaßt, wobei sich dieser Stiel (44) zwischen einem proximalen (40) und einem distalen (46) Ende erstreckt und einen reduzierten mittleren Stielabschnitt (50) in einem Bereich hat, der sowohl vom proximalen als auch vom distalen Ende einen Abstand hat, **dadurch gekennzeichnet,** daß der mittlere Stielabschnitt (50) entlang seiner Länge einen im wesentlichen gleichmäßigen Querschnitt besitzt, um so das Massenträgheitsmomoment an einer bestimmten Stelle entlang der Länge des Stiels (50) zu beeinflussen und so eine optimale Stielflexibilität zu erreichen.

2. Künstliches Gelenk für den Ersatz eines beschädigten natürlichen Gelenkes in einer Skelettstruktur eines Körpers mit
einem becherförmigen Pfannenteil (36), das an einem ersten Knochen (30) des Gelenkes befestigbar ist,
einem Kugelteil (34), das drehbar in das Pfannenteil (36) eingreifen kann,
einem Befestigungsteil (22) mit einem länglichen Stiel (44) zur Befestigung des Kugelteils (34) an einem zweiten Knochen (24) getrennt von dem ersten Knochen, wobei der zweite Knochen ein langer Knochen mit einem Intramedullarkanal (48) ist, der Stiel eine Längsachse besitzt, die allgemein in einer Koronarebene liegt und von dem Intramedullarkanal (48) des zweiten Knochens (24) aufgenommen werden kann, der Stiel sich von einem Proximalende nahe dem Kugelteil bis zu einem Distalende in einem Abstand von dem Kugelteil erstreckt und einen reduzierten mittleren Stielabschnitt in einem Bereich im Abstand sowohl von dem Proximalende als auch von dem Distalende hat, der Stiel (44) einen proximalen (40), mittleren (50) und distalen (46) Abschnitt begrenzt, wobei jeder Abschnitt an jeweiligen proximalen und distalen Enden endet, das Proximalende des proximalen Abschnittes (40) an dem Kugelteil (34) befestigt ist und das Distalende des distalen Abschnittes (46) ein Abschlußende des Stiels begrenzt, und

wobei das Distalende des proximalen Abschnittes (40) und das Proximalende des distalen Abschnitte (46) jeweils direkt an eine sich verjüngende Verbindung mit dem proximalen bzw. distalen Ende des Mittelabschnittes (50) angrenzen,

**dadurch gekennzeichnet,** daß die Querschnittsfläche des Mittelabschnittes (50) über seine Länge einen im wesentlichen gleichmäßigen Querschnitt hat, um so das Massenträgheitsmoment an irgendeiner Stelle entlang der Länge des Stiels (44) zu beeinflussen und so eine optimale Stielflexibilität zu erreichen.

3. Bestandteil nach Anspruch 1 oder Gelenk nach Anspruch 2, bei denen der mittlere Stielabschnitt (50) einen symmetrischen Querschnitt aufweist.

4. Bestandteil nach Anspruch 1 oder Gelenk nach Anspruch 2, bei denen der mittlere Stielabschnitt (50) einen asymmetrischen Querschnitt aufweist.

5. Bestandteil nach Anspruch 1 oder Gelenk nach Anspruch 2, bei denen die Querschnittsfläche des mittleren Stielabschnittes (50) um bis zu 75% der Fläche eines vergleichbaren nichtprofilierten Implantatstieles mit voller Größe reduziert sein kann.

6. Bestandteil nach Anspruch 1 oder Gelenk nach Anspruch 2, die aus einem der Materialien Titan, Titanlegierung, Kobalt-Chrom-Legierung und Verbundmaterialien bestehen.

7. Künstliches Gelenk nach Anspruch 2, bei dem der erste Knochen die Pelvis und bei dem der zweite Knochen die Femur ist und bei dem das Kugelteil und der Stiel Teile eines femoralen Bestandteils einer Hüftprothese sind.

**Revendications**

1. Composant d'une articulation artificielle (20) pour remplacer une articulation naturelle endommagée dans le squelette d'un corps qui comprend une prothèse comportant un premier élément coopérant (22) pouvant être fixé à un premier os long (24) comportant un canal intramédullaire (48) et un second élément coopérant (26) pouvant être fixé à un second os (30), le premier (22) et le second (26) éléments coopérants s'engageant mutuellement et étant relativement mobiles pour permettre le déplacement relatif entre le premier (24) et le second (30) os, le composant comprenant :

une tige allongée (44) comportant un axe longitudinal se trouvant généralement dans un plan coronaire et étant d'un seul tenant avec le premier élément coopérant (22) et pouvant être reçue dans le canal intramédullaire (48) du premier os (24), ladite tige (44) s'étendant entre des extrémités proximale (40) et distale (46) et comportant une section intermédiaire réduite (50) dans une région espacée à la fois de ladite extrémité proximale et de ladite extrémité distale, caractérisé en ce que la section intermédiaire réduite (50) est sensiblement uniforme en section transversale sur sa longueur, de manière à avoir un effet sur le moment d'inertie de masse en n'importe quel emplacement donné le long de ladite tige (44) afin d'obtenir une flexibilité de tige optimale.

2. Articulation artificielle pour remplacer une articulation naturelle endommagée dans le squelette d'un corps comprenant :

une cavité en forme de cuvette (36) pouvant être fixée à un premier os (30) de l'articulation;

une bille (34) pouvant être engagée à rotation avec ladite cavité (36);

un élément de montage (22) comprenant une tige allongée (44) pour fixer ladite bille (34) à un second os (24) séparé du premier os, ledit second os étant un os long comportant un canal intramédullaire (48), ladite tige comportant un axe longitudinal se trouvant généralement dans un plan coronaire et pouvant être reçue dans le canal intramédullaire (48) du premier os (24), ladite tige s'étendant d'une extrémité proximale adjacente à ladite bille à une extrémité distale éloignée de ladite bille, et comportant une section intermédiaire réduite dans une région espacée à la fois de ladite extrémité proximale et de ladite extrémité distale, ladite tige (44) formant des segments proximal (40), intermédiaire (50) et distal (46), chaque segment se terminant à des extrémités proximale et distale respectives, ladite extrémité proximale du segment proximal (40) étant fixée à ladite bille (34) et ladite extrémité distale du segment distal (46) formant une extrémité terminale de ladite tige; et

ladite extrémité distale du segment proximal (40) et l'extrémité proximale du segment distal (46) étant directement adjacentes et formant une jonction conique avec lesdites extrémités proximale et distale du segment intermédiaire (50), respectivement;

caractérisée en ce que la superficie du segment intermédiaire (50) est sensiblement uniforme en section transversale sur sa longueur de manière à avoir un effet sur le moment d'inertie de masse en n'importe quel emplacement donné le long de ladite tige (44) afin d'obtenir une flexibilité de tige optimale.

3. Composant selon la revendication 1 ou articulation selon la revendication 2 où ladite section intermédiaire (50) est symétrique en section transversale.

4. Composant selon la revendication 1 ou articulation selon la revendication 2 où ladite section intermédiaire (50) est asymétrique en section transversale.

5. Composant selon la revendication 1 ou articulation selon la revendication 2 où la superficie en section transversale de ladite section intermédiaire (50) peut être réduite de 75 % par rapport à la superficie d'une tige d'implant comparable, non profilée, grandeur nature.

6. Composant selon la revendication 1 ou articulation selon la revendication 2 qui est composé de titane ou d'alliage de titane ou d'alliage cobalt-chrome et de matériaux composites.

7. Articulation artificielle selon la revendication 2 dans laquelle le premier os est le bassin; et dans laquelle le second os est le fémur; et dans laquelle ladite bille et ladite tige sont des parties d'un composant fémoral d'une prothèse pour la hanche.

FIG. I

FIG. 2

FIG. 2A

FIG. 4

FIG. 3

RELATIVE STIFFNESS (BENDING)

STEM DIAMETER (mm)

# FIG. 5

$$8.414 \left( \frac{1}{Nmm^2} \times 10^{-10} \right)$$

$$739.0 \left( \frac{1}{Nmm^2} \times 10^{-10} \right)$$

$$24.89 \left( \frac{1}{Nmm^2} \times 10^{-10} \right)$$

EP 0 358 399 B1

FIG. 6

11